# EUROPÄISCHE PATENTSCHRIFT

(11) **EP 3 366 256 B1**
(45) Veröffentlichungstag und Bekanntmachung des Hinweises auf die Patenterteilung: **13.05.2020**
(21) Anmeldenummer: 18157381.7
(22) Anmeldetag: 19.02.2018
(51) Int. Cl.: A61C 1/08, A61B 17/17, A61C 8/02

(54) **VORRICHTUNG ZUM FÜHREN EINER DENTALCHIRURGISCHEN HOHLFRÄSE**
DEVICE FOR GUIDING A DENTAL SURGERY HOLLOW MILL
DISPOSITIF DE GUIDAGE D'UNE FRAISE CREUSE DE CHIRURGIE DENTAIRE

(30) Priorität: 22.02.2017 DE 102017202817
(43) Veröffentlichungstag der Anmeldung: 29.08.2018
(73) Patentinhaber: Zastrow, Frank, 69120 Heidelberg (DE)
(72) Erfinder: Zastrow, Frank, 69120 Heidelberg (DE)
(74) Vertreter: Patent- und Rechtsanwälte Ullrich & Naumann

(56) Entgegenhaltungen:
- WO-A1-2016/188522
- DE-A1-102012 102 255
- US-A1- 2016 106 513
- US-A1- 2016 157 960

## Beschreibung

Die vorliegende Erfindung betrifft eine Vorrichtung zum Führen einer dentalchirurgischen Hohlfräse bei einer Knochenentnahme.

In diesem Zusammenhang wird die US 2016/106513 A1 genannt.

Chirurgisch tätige Zahnärzte, Oralchirurgen und Kieferchirurgen stehen häufig vor der Herausforderung, dass durch Knochenatrophie, durch Unfälle, durch Parodontitis oder durch Zahnextraktion Knochen in der Mundhöhle verloren gegangen ist.

Wenn Zahnimplantate zum Einsatz neuer Zähne geplant werden, so ist es wichtig, dass diese Knochendefizite vorher oder simultan mit der Implantatsetzung aufgebaut werden, damit die Zahnimplantate wieder ein neues Fundament und eine stabile Abstützung im Knochen haben.

Autologer, patienteneigener Knochen gilt dabei nach wie vor als Goldstandard bei knochenaufbauenden Eingriffen. Dies liegt an den Eigenschaften des Knochens, da autologer Knochen sowohl osteogene, osteoinduktive sowie osteokonduktive Eigenschaften miteinander vereint. Das bedeutet, der Knochen hat die Potenz, eigenes Knochengewebe zu bilden, Gefäße zu bilden und wirkt zudem als Leitstruktur für neu gebildeten Knochen. Knochenersatzmaterial hat im Gegensatz zu autologem Knochen keine biologische Potenz und wirkt lediglich osteokonduktiv, das heißt, es wirkt auch als Leitschiene.

Beim Arbeiten mit autologem Knochen sind verschiedene Verfahren vorbekannt. Bei größeren Knochendefiziten bieten sich als zweite intraorale Entnahmestelle grundsätzlich zahnlose Knochenareale an, alternativ der Tuber maxillae, die Spina nasalis anterior, der Gaumen, der Bereich der Kieferhöhlenwand im Oberkiefer oder aber der Unterkiefer, da dieser eher kortikaler Natur ist und die Knochenqualität als sehr gut und stabil gilt. Im Unterkiefer gibt es verschiedene Knochenentnahmestellen, so beispielsweise wiederum zahnlose Areale, das Kinn oder den retromolare Bereich.

Die Knochenentnahme kann hierbei mit verschiedenen Instrumenten erfolgen. Das Konzept der Knochenentnahme ist dabei meist ähnlich.

Entweder werden mit einer sogenannten Lindemannfräse oder mit einem Piezosurgerygerät oder aber mit einer kleinen Säge drei bis vier Sollbruchstellen geschaffen und der Block im Nachhinein mit einem Meisel oder einem anderen Instrument herausgebrochen. Dabei ist nachteilig, dass bei einer Knochenentnahme mehr oder weniger große Gewaltanwendung auf den Kiefer nötig ist. Daher wird dieser Eingriff teilweise vom Arzt gescheut, auch aus dem Grund, da dieses Heraushämmern bzw. -brechen des Knochenblockes aus der jeweiligen Region auch für den Patienten unangenehm ist.

Zahnärzte sind aufgrund Ihres Berufes rotierende Instrumente und Bohrer gewohnt. So hat sich auch die sogenannte Trepanfräse bzw. Hohlfräsen etabliert, die an ein Handstück angesteckt wird und einen Kopf aufweist, der als Hohlzylinder ausgebildet ist. An dem distalen Rand des Kopfes sind Zähne zur spanenden Bearbeitung des Knochens ausgebildet. Trepanfräsen bzw. Hohlfräsen werden beispielsweise zur Implantatbettaufbereitung genutzt und weisen einen Durchmesser von ca. 3 mm bis 4 mm auf. Mit Hilfe dieser Fräsen werden äußerst kleine und schmale Bohrzylinder entnommen, die nur bedingt zum Knochenaufbau geeignet sind.

Bei den voranstehend genannten Vorrichtungen müssen zur Knochenentnahme ca. drei bis vier Schnitte bzw. Sollbruchstellen angelegt werden, um das benötigte Knochenstück zu erhalten. Dies bedingt eine starke Belastung für den Patienten und erfordert großes handwerkliches Geschick durch den Operateur. Insbesondere ist dabei zu beachten, dass das umliegende Weichgebewebe, beispielsweise Wange oder Lippe, durch das chirurgische Werkzeug nicht verletzt wird.

Der vorliegenden Erfindung liegt daher die Aufgabe zugrunde, eine Vorrichtung anzugeben, so dass mit konstruktiv einfachen Mitteln eine zuverlässige und für den Patienten schonende Knochenentnahme möglich ist. Des Weiteren soll ein Verfahren zur Herstellung einer solchen Vorrichtung angegeben werden.

Erfindungsgemäß wird die voranstehende Aufgabe durch die Merkmale des Anspruchs 1 gelöst. Danach ist eine Vorrichtung zum Führen einer dentalchirurgischen Hohlfräse bei einer Knochenentnahme angegeben, mit einer im Bereich einer Entnahmestelle festlegbaren Schablone und mindestens einem an der Schablone angeordneten Führungsmittel, wobei das Führungsmittel die Hohlfräse derart führt, dass lediglich ein Teil des distalen Randes der Hohlfräse mit dem Knochen in Kontakt bringbar ist und wobei die Hohlfräse durch das Führungsmittel seitlich am Knochen entlangführbar ist, so dass ein kreissegmentförmiges Knochenstück auslösbar ist.

In erfindungsgemäßer Weise ist zunächst erkannt worden, dass die zugrundeliegende Aufgabe durch eine Schablone mit einem Führungsmittel zum Führen einer dentalchirurgischen Hohlfräse in raffinierter Weise gelöst werden kann. Die Schablone ist dabei im Bereich der Entnahmestelle festlegbar. An der Schablone ist ein Führungsmittel angeordnet, das die Hohlfräse in einem exakt definierten Winkel zu der Entnahmestelle führt, so dass die rotierende Hohlfräse lediglich mit einem Teil des distalen Randes mit dem Knochen zur Knochenentnahme in Kontakt bringbar ist. Im Konkreten kann dabei die Hohlfräse seitlich am Knochen entlang geführt werden, so dass ein kreissegmentförmiges Knochenstück ausgelöst wird. Somit ist gewährleistet, dass die Hohlfräse von dem Operateur in einer geführten Bewegung, insbesondere unter einem exakten Winkel, in die Entnahmestelle einbringbar ist.

In weiter erfindungsgemäßer Weise ist erkannt worden, dass sich entgegen eines Vorurteils der Fachwelt eine Hohlfräse nicht nur zur Entnahme von kleinen Bohrzylindern eignet. Vielmehr lässt sich eine entsprechend ausgebildete Hohlfräse dazu nutzen, das benötigte Knochenstück bzw. Knochensegment aus dem Knochen "herauszuschälen". Aufgrund des an der Schablone angeordneten Führungsmittels ist die Hohlfräse in einer eben solchen Weise mit dem Knochen der Entnahmestelle in Kontakt bringbar. Mit anderen Worten ist lediglich ein Kreisbogen des distalen Randes des Kopfes der Hohlfräse mit dem Knochen in Kontakt bringbar und dient somit als effektiver Wirkbereich. Im Gegensatz zu den aus dem Stand der Technik bekannten Instrumenten bzw. Werkzeugen müssen keine drei bis vier Schnitte bzw. Sollbruchstellen angelegt werden. Es entsteht vielmehr lediglich eine einzige Sollbruchstelle, ein Heraushämmern des Knochens ist nicht notwendig. Der entstehende Block ist sodann ohne größere Gewalteinwirkung herauslösbar bzw. luxierbar, was für den Patienten im Vergleich zu den bekannten Vorrichtungen und Techniken schonender und angenehmer ist. Folglich ist die Belastung durch den Eingriff für den Patienten minimal.

An dieser Stelle sei darauf hingewiesen, dass es sich bei dem "distalen Rand" der Hohlfräse um den Bereich des hohlzylinderförmigen Kopfes der Hohlfräse handelt, mit dem der Knochen bearbeitet, d.h. gefräst, geschliffen bzw. durchgetrennt wird. Der Begriff "Hohlfräse" ist im weitesten Sinne zu verstehen, kann diese bspw. Sägezähne und/oder eine Diamantierung im Wirkbereich aufweisen und an ein zahnärztliches Handstück ankoppelbar sein, um in Rotation versetzt zu werden. Die "Entnahmestelle" ist der Bereich des Knochens, aus dem das benötigte Knochenstück herausgetrennt wird. Das "Knochendefizit" ist der Bereich des Knochens, der durch das entnommene Knochensegment wieder aufgebaut werden soll.

Im Konkreten kann die Schablone form- und/oder kraftschlüssig im Oberkiefer und/oder im Unterkiefer festlegbar ausgebildet sein. Dabei ist denkbar, dass die Schablone zumindest teilweise als Schiene ausgebildet ist und/oder über Haltestifte bzw. Schrauben fixierbar ist. Im Allgemeinen kann die Schablone knochengetragen und/oder zahngetragen und/oder schleimhautgetragen realisiert sein. Auch ist denkbar, dass die Schablone über eine externe Fixiereinrichtung, die beispielsweise am Kopf des Patienten anliegt, festgelegt ist.

Um an mehreren Entnahmestellen Knochen zu entnehmen, können an der Schablone mehrere Führungsmittel ausgebildet sein. Dadurch ist die Hohlfräse durch die Führungsmittel hindurch an unterschiedlichen Entnahmestellen mit dem Knochen in Kontakt bringbar. Somit ist es mit konstruktiv einfachen Mitteln möglich, eine größere Menge von Knochen zu entnehmen.

In vorteilhafter Weise kann das Führungsmittel die Hohlfräse derart umgeben, dass das die Entnahmestelle umliegende Gewebe nicht mit der Hohlfräse in Kontakt kommt. Somit dient das Führungsmittel nicht nur dazu, die Hohlfräse in einer definierten Position mit dem Knochen in Kontakt zu bringen, sondern auch als Schutzelement, welches das umliegende Gewebe gegenüber der Hohlfräse abschirmt.

Weiterhin ist denkbar, dass das Führungsmittel als Führungshülse ausgebildet sein kann, in die die Hohlfräse einbringbar ist. Die Führungshülse kann eine seitliche Öffnung aufweisen, so dass ein Teil des distalen Randes der Hohlfräse durch die Öffnung der Führungshülse hindurch mit dem Knochen in Kontakt bringbar ist. Durch die Ausgestaltung des Führungsmittels als Führungshülse wird das umliegende Weichgewebe in optimaler Weise vor Verletzungen durch die Hohlfräse geschützt. An dieser Stelle sei darauf hingewiesen, dass der Begriff Führungshülse im weitesten Sinne zu verstehen ist. Die Führungshülse muss bspw. nicht zwangsweise rund bzw. als Teil eines Kreiszylinders ausgebildet sein, kann nämlich auch eine zumindest bereichsweise eckige Geometrie aufweisen. In besonders vorteilhafter Weise kann die Führungshülse an ihrem Endbereich geschlossen sein, weist somit einen Boden auf. Dadurch ist nochmals sichergestellt, dass das umliegende Gewebe geschützt wird. Bei einer Ausgestaltung des Führungsmittels als Führungshülse ist es von Vorteil, wenn die Hohlfräse ein mit dem Innendurchmesser der Führungshülse korrespondierendes Distanzelement aufweist, das drehbar mit der Hohlfräse gekoppelt ist. Dadurch ist die Hohlfräse in der Führungshülse drehbar und es wird vermieden, dass der distale Rand der Hohlfräse mit der Führungshülse in Kontakt kommt.

Alternativ oder zusätzlich kann das Führungsmittel eine, insbesondere im Wesentlichen zylinderförmige, Aufnahme für ein korrespondierendes Führungselement der Hohlfräse aufweisen. Bei dem Führungselement der Hohlfräse kann es sich bspw. um einen von dem Boden des Kopfes der Hohlfräse in Axialrichtung verlaufenden Führungszapfen handeln. Die Aufnahme kann als mit dem Führungszapfen korrespondierende Hülse bzw. Röhrchen ausgebildet sein. Des Weiteren ist denkbar, dass das Führungsmittel der Schablone mit einem korrespondierenden Führungselement der Hohlfräse in Eingriff bringbar ist. Beispielsweise kann ein mit der Hohlfräse drehbar gekoppeltes Führungselement, insbesondere eine Schiene, vorgesehen sein, das mit dem Führungsmittel, bspw. einer Schiene, korrespondiert.

Um zu gewährleisten, dass die Hohlfräse nicht zu tief in den Knochen eindringt, kann an dem Führungsmittel ein Tiefenanschlag im Sinne eines Tiefenstopps für die Hohlfräse ausgebildet sein.

In vorteilhafter Weise kann das Führungsmittel, insbesondere die Führungshülse, lösbar mit der Schablone verbunden sein. Beispielsweise können an dem Führungsmittel, insbesondere der Führungshülse, und an der Schablone miteinander korrespondierende Kopplungselemente vorgesehen sein, über die das Führungsmittel im gewünschten Winkel an der Schablone festlegbar ist. Die korrespondierenden Kopplungselemente können beispielsweise als Matrize und Patrize ausgebildet sein. Weiterhin ist es von Vorteil, das Führungsmittel, insbesondere die Führungshülse, aus Metall herzustellen, so dass diese für einen mehrfachen Gebrauch wiederaufbereitet werden kann. In weiter vorteilhafter Weise können an unterschiedlichen Bereichen der Schablone Kopplungselemente vorgesehen sein, so dass das Führungsmittel an unterschiedlichen Bereichen des Grundelements im jeweils benötigten Winkel an der Schablone festlegbar ist und somit an unterschiedlichen Entnahmestellen eine Knochenentnahme erfolgen kann.

Weiterhin ist es denkbar, dass das Führungsmittel, insbesondere die Führungshülse, einteilig mit der Schablone ausgestaltet ist bzw. ein fester Bestandteil der Schablone ist, bspw. mit dieser verklebt, verschweißt etc. ist. Bei einer einteiligen Ausgestaltung können mehrere Führungsmittel, insbesondere Führungshülsen, an unterschiedlichen Bereichen des Grundelements vorgesehen sein.

Gemäß einer weiteren Ausgestaltung kann die Schablone ein Haltemittel aufweisen, um das entnommene Knochenstück im Bereich eines aufzubauenden Knochendefizits zu halten. Das Knochenstück kann im Konkreten form- und/oder kraftschlüssig mit der Schablone verbindbar sein, beispielsweise in dieses eingeklipst bzw. eingespannt werden. Dadurch ist es dem Operateur möglich, das Knochenstück in der gewünschten Position im Bereich des Knochendefizits zu fixieren, insbesondere mit dem aufzubauenden Kieferknochen zu verschrauben.

In besonders vorteilhafter Weise kann das Führungsmittel, insbesondere die Führungshülse, derart angeordnet bzw. ausgebildet sein, dass eine Hohlfräse mit der erfindungsgemäßen Schablone verwendbar ist, deren Kopf einen Außendurchmesser von 5 mm bis 11 mm, vorzugsweise von 6 mm bis 8 mm, und/oder einer Tiefe von 6 mm bis 17 mm, insbesondere 9 mm bis 14 mm, aufweist. In weiter vorteilhafter Weise kann das Führungsmittel, insbesondere die Führungshülse, derart angeordnet bzw. ausgebildet sein, dass der mit dem Knochen in Kontakt bringbare Kreisbogen des distalen Rands der Hohlfräse ein Kreissegment bildet, das eine Segmenthöhe von 2,0 mm bis 3,5 mm, vorzugsweise von 2,2 mm bis 2,5 mm, aufweist.

Die zugrunde liegende Aufgabe wird des Weiteren durch ein Führungsmittel gemäß Anspruch 10 gelöst. Danach ist ein Führungsmittel für eine Vorrichtung nach einem der Ansprüche 1 bis 9 angegeben, mit einem Kopplungselement zur vorzugsweise lösbaren Befestigung an einer Schablone. Bei dem Kopplungselement kann es sich auch um einen definierten Bereich des Führungsmittels handeln, der mit der Schablone verklebt oder verschraubt ist oder anderweitig an dieser befestigbar ist. Weiterhin kann es sich bei dem Kopplungselement um eine Matrize bzw. Patrize handeln, die mit einer korrespondierenden Patrize bzw. Matrize der Schablone in Eingriff bringbar ist.

Weiterhin wird die zugrunde liegende Aufgabe durch eine Schablone nach Anspruch 11 gelöst. Danach ist eine Schablone für eine Vorrichtung nach einem der Ansprüche 1 bis 9 angegeben, mit einem Kopplungselement zur vorzugsweise lösbaren Befestigung eines Führungsmittels. Bei dem Kopplungselement kann es sich auch um einen definierten Bereich der Schablone handeln, mit dem das Führungsmittel verklebt oder verschraubt ist oder anderweitig an diesem befestigbar ist. Weiterhin kann es sich bei dem Kopplungselement um eine Matrize bzw. Patrize handeln, die mit einer korrespondierenden Patrize bzw. Matrize des Führungsmittels in Eingriff bringbar ist.

Das Führungsmittel und/oder die Schablone können gemäß des in den Ansprüchen 1 bis 9 beschriebenen Führungsmittels bzw. der dort beschriebenen Schablone ausgestaltet sein und die voranstehend in Bezug auf dieses Führungsmittel bzw. diese Schablone genannten Merkmale und Vorteile aufweisen. Des Weiteren können das Führungsmittel gemäß Anspruch 10 sowie die Schablone gemäß Anspruch 11 einzelne oder sämtliche Merkmale des in der nachfolgenden Figurenbeschreibung beschriebenen Führungsmittels bzw. der beschriebenen Schablone aufweisen.

Des Weiteren wird die zugrundeliegende Aufgabe durch das Verfahren mit den Merkmalen des Anspruchs 12 gelöst. Danach ist ein Verfahren zur Herstellung einer Vorrichtung nach einem der Ansprüche 1 bis 9 mit folgenden Verfahrensschritten angegeben:
Durchführen eines dreidimensionalen, bildgebenden Verfahrens der Entnahmestelle und ggf. des Knochendefizits, insbesondere des Ober- und/oder Unterkiefers, zur Gewinnung von dreidimensionalen Daten,
Erstellung eines dreidimensionalen Computermodells der Entnahmestelle und ggf. des Knochendefizits anhand der dreidimensionalen Daten,
Erzeugung der Schablone auf Grundlage des dreidimensionalen Computermodells.

In beispielhafter Weise wird zunächst ein dreidimensionales, bildgebendes Verfahren an der Entnahmestelle und ggf. an dem Knochendefizit durchgeführt. Bei der Entnahmestelle kann es sich insbesondere um den Ober- und/oder Unterkiefer handeln. Anhand der durch dieses Verfahren gewonnenen dreidimensionalen Daten wird in weiter beispielhafter Weise ein dreidimensionales Computermodell der Entnahmestelle und ggf. des Knochendefizits erzeugt. Dies hat den Vorteil, dass die Knochenentnahme virtuell planbar ist, insbesondere unter Berücksichtigung der individuellen Anatomie des Patienten, beispielsweise dem Verlauf von Nerven, Wurzelkanälen etc. Anhand dieses dreidimensionalen Computermodells wird sodann in weiter beispielhafter Weise die patientenspezifische Schablone geplant bzw. erzeugt. Auf Grundlage des dreidimensionalen Computermodells kann des Weiteren die Entnahmestelle und somit die Positionierung des Führungsmittels exakt bestimmt werden. Beispielsweise kann die Schablone mittels eines Rapid-Prototyping-Verfahrens, insbesondere einem Stereolithografie-Verfahren oder einem 3D-Druckverfahren, hergestellt werden. Jegliche hierfür geeigneten und insbesondere in der Zahntechnik bekannten Verfahren können Anwendung finden. Sofern das Führungsmittel einteilig mit der Schablone ausgebildet ist, kann dieses im gleichen Arbeitsschritt zusammen mit der Schablone erzeugt werden, nämlich anhand des dreidimensionalen Computermodells. Sofern das Führungsmittel kein integraler Bestandteil der Schablone ist, können die Kopplungselemente auf Grundlage des dreidimensionalen Computermodells an der Schablone erzeugt werden, so dass das Führungsmittel mit korrespondierenden Kopplungselementen in der gewünschten, definierten Position an der Schablone festlegbar ist.

In vorteilhafter Weise kann als dreidimensionales, bildgebendes Verfahren eine Computertomografie und/oder eine Magnetresonanztomografie und/oder eine digitale Volumentomografie angewandt werden.

Die Offenbarung betrifft des Weiteren eine dentalchirurgische Hohlfräse, wobei ein Distanzelement drehbar mit der Hohlfräse gekoppelt ist. Dadurch wird erreicht, dass die Hohlfräse nicht mit dem Führungsmittel in Kontakt bringbar ist. Die Hohlfräse kann in vorteilhafter Weise die in der nachfolgenden Figurenbeschreibung und den entsprechenden Figuren offenbarten Merkmale bzw. einzelne dieser Merkmale aufweisen.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Erfindung anhand der Zeichnung zu verweisen, anhand der auch ein Verfahren erläutert wird. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen, perspektivischen Darstellung ein erstes Ausführungsbeispiel einer erfindungsgemäßen Vorrichtung,
- Fig. 2: in einer weiteren schematischen, perspektivischen Darstellung das Ausführungsbeispiel gemäß Fig. 1,
- Fig. 3: in einer schematischen, perspektivischen Darstellung ein weiteres Ausführungsbeispiel einer erfindungsgemäßen Vorrichtung,
- Fig. 4: in einer weiteren schematischen, perspektivischen Darstellung das Ausführungsbeispiel gemäß Fig. 3,
- Fig. 5: in einer schematischen, perspektivischen Darstellung ein weiteres Ausführungsbeispiel einer erfindungsgemäßen Vorrichtung,
- Fig. 6: in einer weiteren schematischen, perspektivischen Darstellung das Ausführungsbeispiel gemäß Fig. 5,
- Fig. 7: in einer schematischen, perspektivischen Darstellung ein weiteres Ausführungsbeispiel einer erfindungsgemäßen Vorrichtung,
- Fig. 8: einen vergrößerten Teilausschnitt aus Fig. 7,
- Fig. 9: in einer schematischen, perspektivischen Darstellung ein weiteres Ausführungsbeispiel einer erfindungsgemäßen Vorrichtung,
- Fig. 10: in einer weiteren schematischen, perspektivischen Darstellung das Ausführungsbeispiel gemäß Fig. 9,
- Fig. 11: in einer weiteren schematischen, perspektivischen Darstellung das Ausführungsbeispiel gemäß Fig. 9,
- Fig. 12: in einer schematischen, perspektivischen Explosionsdarstellung ein weiteres Ausführungsbeispiel einer erfindungsgemäßen Vorrichtung,
- Fig. 13: in einer weiteren schematischen, perspektivischen Darstellung das Ausführungsbeispiel gemäß Fig. 12,
- Fig. 14: in einer weiteren schematischen, perspektivischen Darstellung das Ausführungsbeispiel gemäß Fig. 12,
- Fig. 15: in einer schematischen, perspektivischen Darstellung ein weiteres Ausführungsbeispiel einer erfindungsgemäßen Vorrichtung,
- Fig. 16: in einer weiteren schematischen, perspektivischen Darstellung das Ausführungsbeispiel gemäß Fig. 15,
- Fig. 17: in einer weiteren schematischen, perspektivischen Darstellung das Ausführungsbeispiel gemäß Fig. 15,
- Fig. 18: in einer schematischen, perspektivischen Darstellung ein weiteres Ausführungsbeispiel einer erfindungsgemäßen Vorrichtung,
- Fig. 19: in einer weiteren schematischen, perspektivischen Darstellung das Ausführungsbeispiel gemäß Fig. 18, und
- Fig. 20: in einer weiteren schematischen, perspektivischen Darstellung das Ausführungsbeispiel gemäß Fig. 18.

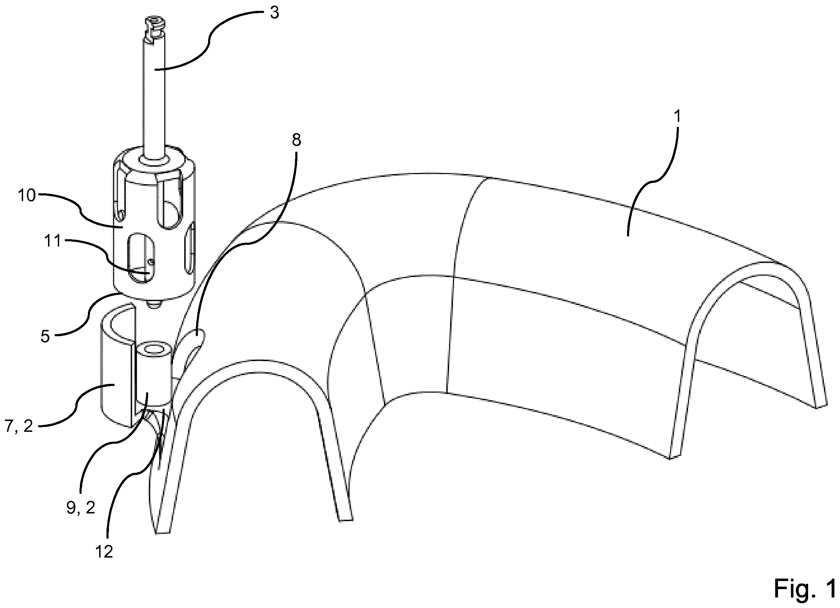

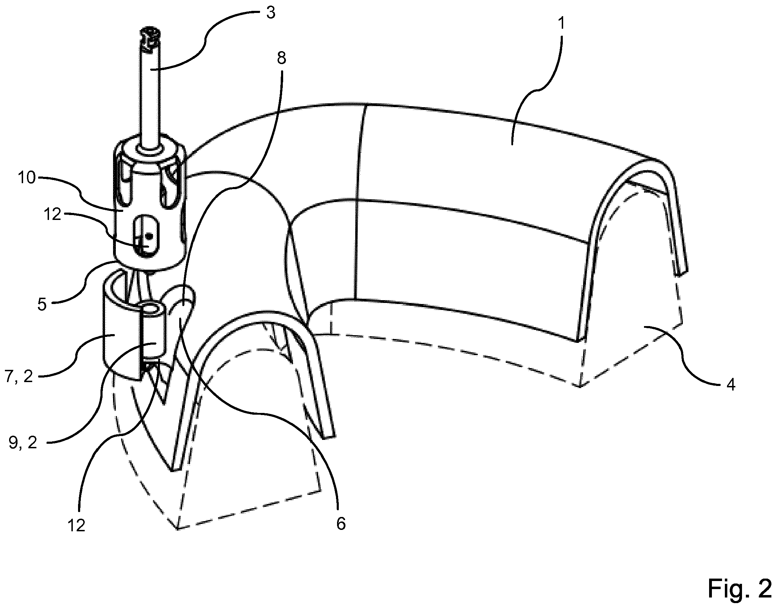

In den Fig. 1 und 2 ist ein erstes Ausführungsbeispiel einer erfindungsgemäßen Vorrichtung dargestellt. Die Vorrichtung weist eine Schablone 1 auf, an der ein Führungsmittel 2 für eine Hohlfräse 3 ausgebildet ist. In Fig. 2 ist schematisch ein Kieferknochen 4 dargestellt, auf bzw. an dem die Schablone 1 angeordnet ist. Es wird darauf hingewiesen, dass die Schablone 1 nicht zwangsweise auf dem Kieferknochen 4, sondern alternativ oder zusätzlich an den vorhandenen Zähnen, dem Zahnfleisch etc. anliegen kann. Dabei ist deutlich zu erkennen, dass das Führungsmittel 2 derart an der Schablone 1 vorgesehen ist, dass lediglich ein Teil des distalen Randes 5 der Hohlfräse 3 in einer definierten Lage mit dem Knochen 4 an der Entnahmestelle 6 in Kontakt bringbar ist. Im Konkreten ist das Führungsmittel 2 derart ausgebildet, dass die Hohlfräse 3 seitlich an dem Kieferknochen 4 entlang geführt wird.

Das Führungsmittel 2 ist als Führungshülse 7 ausgebildet, wobei die Führungshülse 7 nicht vollständig geschlossen ist. Die Führungshülse 7 dient neben der Führung der Hohlfräse 3 zusätzlich als Schutz des umliegenden Gewebes und kann auch geschlossen ausgebildet sein, wobei eine Öffnung 8 vorgesehen sein muss, durch welche die Hohlfräse 3 mit dem Knochen 4 in Kontakt bringbar ist. Des Weiteren ist den Innendurchmesser der Führungshülse 7 vorzugsweise derart zu dimensionieren, dass die rotierenden Hohlfräse 3 nicht mit der Führungshülse 7 in Kontakt kommt.

Des Weiteren umfasst das Führungsmittel 2 eine zylinderförmige Aufnahme 9, die als Kreiszylinder bzw. Röhrchen realisiert ist und zum Einbringen eines innerhalb des Kopfes 10 der Hohlfräse 3 verlaufenden Führungselements 11 bzw. Führungszapfens dient. Die Aufnahme 9 wirkt somit als Zwangsführung für die Hohlfräse 3. An dieser Stelle sei darauf hingewiesen, dass das Führungsmittel 2 nicht zwangsweise die Führungshülse 7 aufweisen muss, sondern lediglich durch die Aufnahme 9 realisiert sein kann.

Auch kann das Führungsmittel 2 lösbar mit der Schablone 1 ausgestaltet sein. Des Weiteren ist ein Tiefenanschlag 12 ausgebildet, der als Tiefenstopp dient, so dass die Hohlfräse 3 lediglich über eine definierte Länge in den Kieferknochen 4 einbringbar ist.

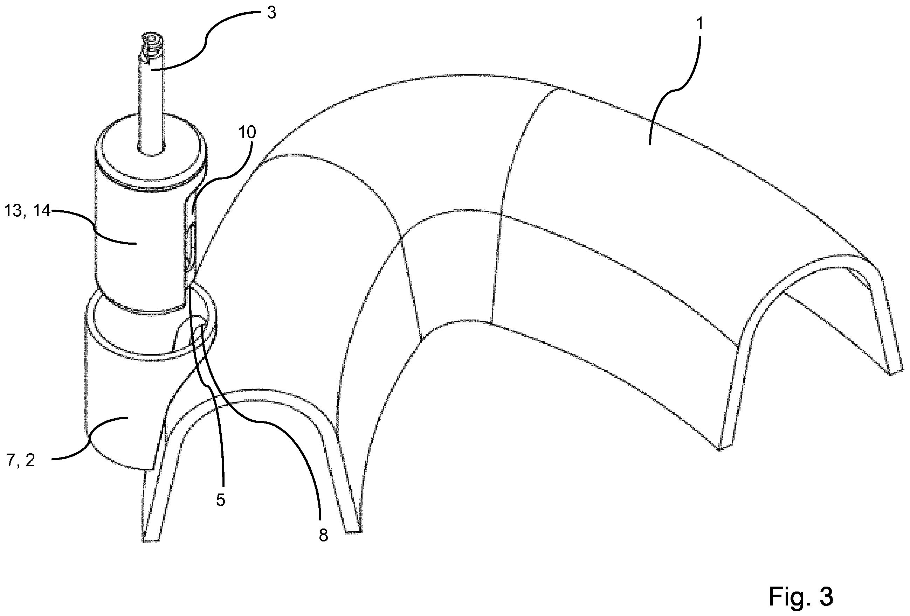

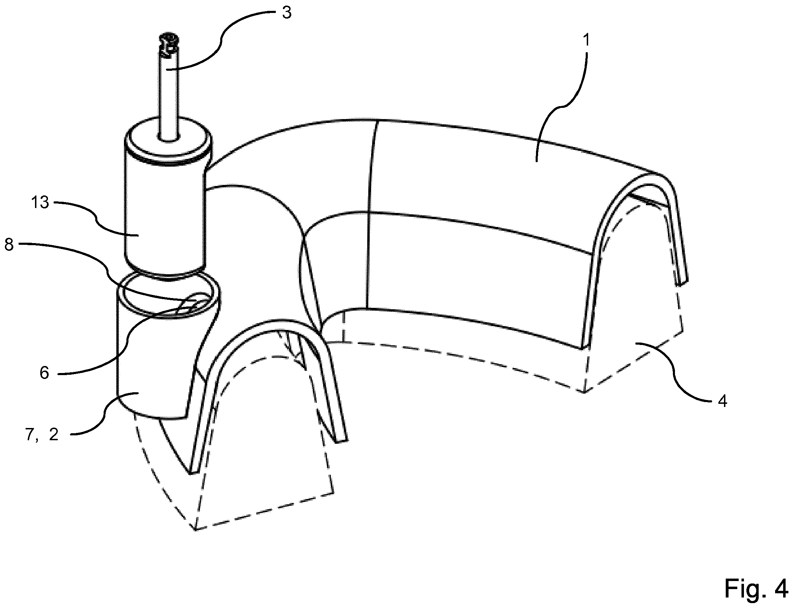

Die Fig. 3 und 4 zeigen ein weiteres Ausführungsbeispiel einer erfindungsgemäßen Vorrichtung. Dabei ist das Führungsmittel 2 als in Richtung des umliegenden Gewebes geschlossene Führungshülse 7 ausgebildet ist, wobei die Führungshülse 7 in Richtung der Entnahmestelle 6 seitlich geöffnet ist. Somit kann ein Teil des distalen Rands 5 den Knochen 4 bearbeiten. In dem hier dargestellten Ausführungsbeispiel ist die Führungshülse 7 einteilig mit dem Grundelement 1 ausgebildet. Die Führungshülse 7 kann jedoch Kopplungselemente aufweisen, über welche sie lösbar mit der Grundelement 1 zu verbinden ist.

Die Hohlfräse 3 ist drehbar mit einer Schutzeinrichtung 13 gekoppelt und gemeinsam mit der Schutzeinrichtung 13 in die Führungshülse 7 einbringbar. Die Schutzeinrichtung 13 weist eine seitliche Öffnung auf und dient als Distanzelement 14, so dass der Kopf 10 nicht mit der Führungshülse 7 bzw. der Schablone 1 in Kontakt kommt. Entgegen der Darstellung muss die Führungshülse 7 nicht zwangsweise geschlossen ausgebildet sein. Es ist ausreichend, wenn die Führungshülse 7 die Schutzeinrichtung 13 lediglich teilweise umgibt. Des Weiteren entspricht das Ausführungsbeispiel gemäß den Fig. 3 und 4 dem in den Fig. 1 und 2 gezeigten Ausführungsbeispiel, so dass zur Vermeidung von Wiederholungen auf die voranstehenden Ausführungen verwiesen wird.

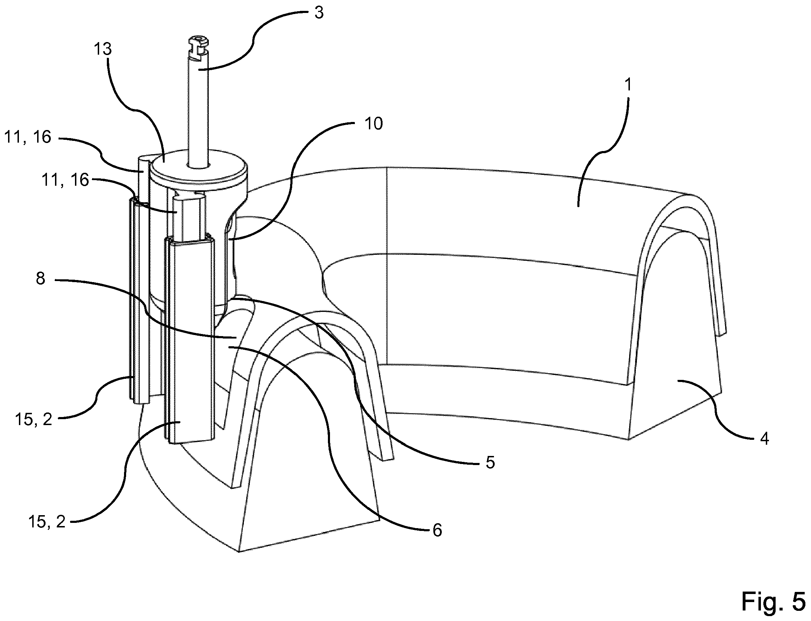

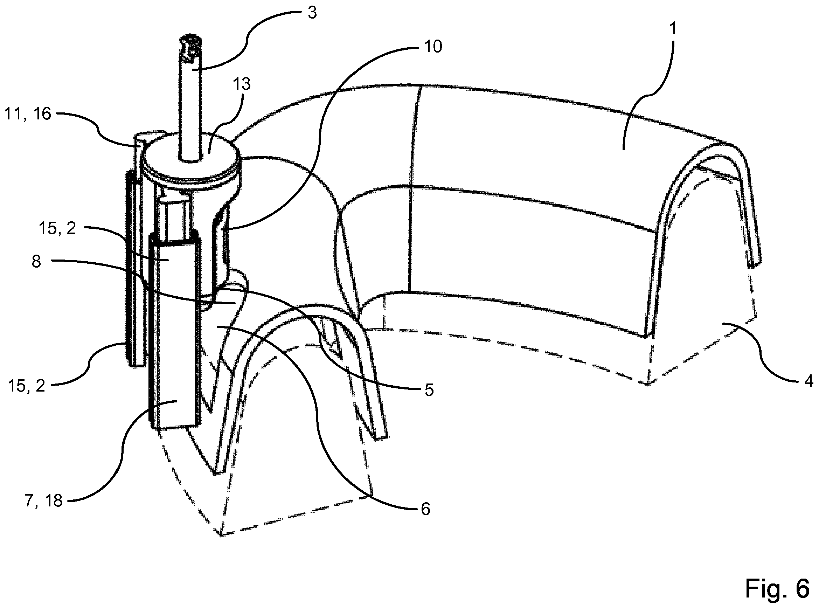

In den Fig. 5 und 6 ist ein weiteres Ausführungsbeispiel einer erfindungsgemäßen Vorrichtung dargestellt. Dabei ist das Führungsmittel 2 der Schablone 1 durch zwei Schienen 15 bzw. Matrizen realisiert. Die Hohlfräse 3 ist mit einer Schutzeinrichtung 13 drehbar gekoppelt, wobei an der Schutzeinrichtung 13 Führungselemente 11 als Schienen 16 bzw. Patrizen ausgebildet sind, die mit den Schienen 15 der Schablone 1 korrespondieren. Somit ist die Hohlfräse 3 über die Schienen 15, 16 geführt. Ansonsten entspricht die Schutzeinrichtung 13 der in den Fig. 3 und 4 dargestellten Schutzeinrichtung 13.

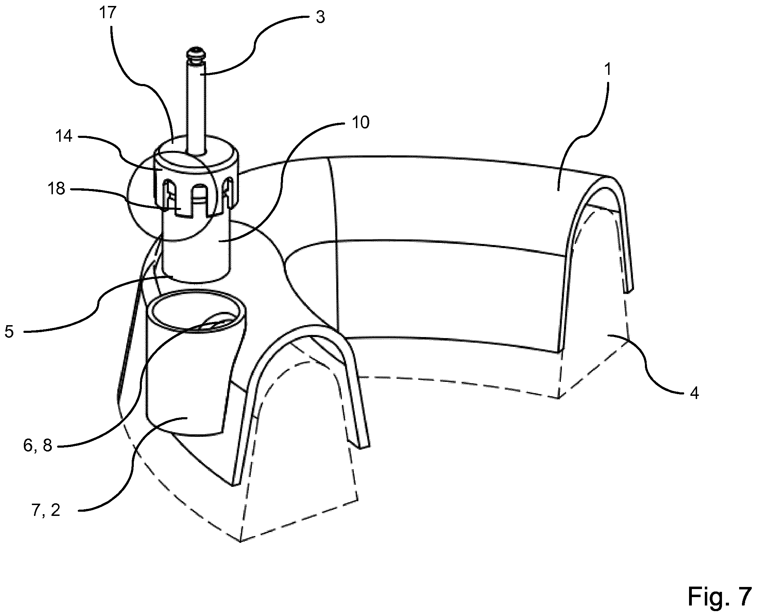

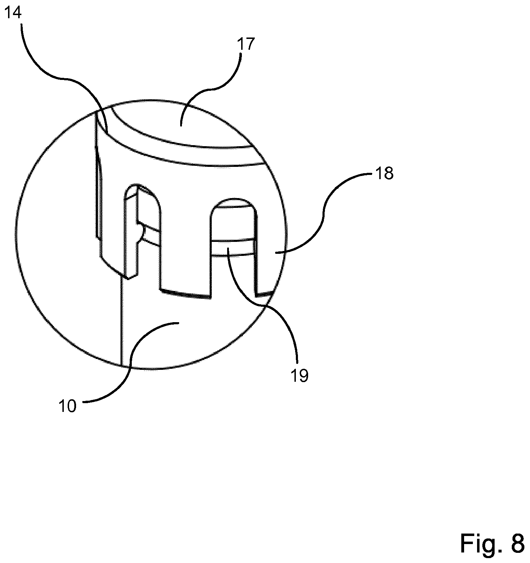

Das in den Fig. 7 und 8 dargestellte Ausführungsbeispiel der erfindungsgemäßen Vorrichtung entspricht der in den Fig. 3 und 4 gezeigten Vorrichtung, so dass zur Vermeidung von Wiederholungen auf die Figurenbeschreibung von Fig. 3 und 4 verwiesen wird. Die Hohlfräse 3 ist mit einem Distanzelement 14 drehbar verbunden. Der Außendurchmesser des Distanzelements 14 entspricht in etwa dem Innendurchmesser der Führungshülse 7. Durch das Distanzelement 14 wird verhindert, dass der Kopf 10 mit der Führungshülse 7 in Kontakt kommt. Das Distanzelement 14 weist ein Bodenelement 17 auf, an dem mehrere Fixierelemente 18 ausgebildet sind. Die Fixierelemente 18 greifen in eine Nut 19 des Kopfes 10 ein. Das Distanzelement 14 umgibt den Kopf 10 somit bereichsweise und ist drehbar mit diesem gekoppelt. Durch die Führungshülse 7 ist das umliegende Gewebe ideal vor Verletzungen durch die Hohlfräse 3 geschützt. Dabei können die Fixierelemente 18 ggf. weiter in Richtung des distalen Rands 5 der Hohlfräse 3 ragen, wobei mindestens ein Fixierelement 18, vorzugweise 3 Fixierelemente 18, ausgebildet sind. Wesentlich ist, dass die Fixierelemente 18 sich weit genug über den Kopf 10 hinweg erstrecken, so dass die Hohlfräse 3 sicher innerhalb der Führungshülse 7 geführt ist.

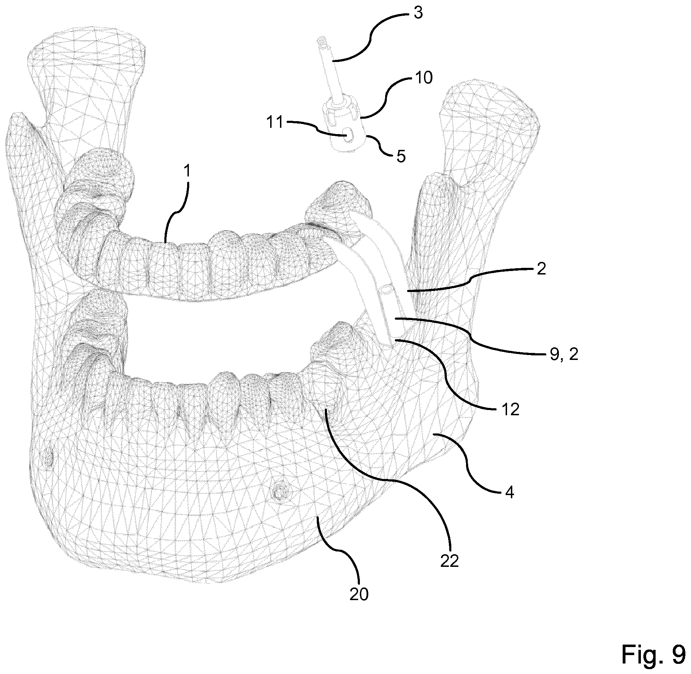

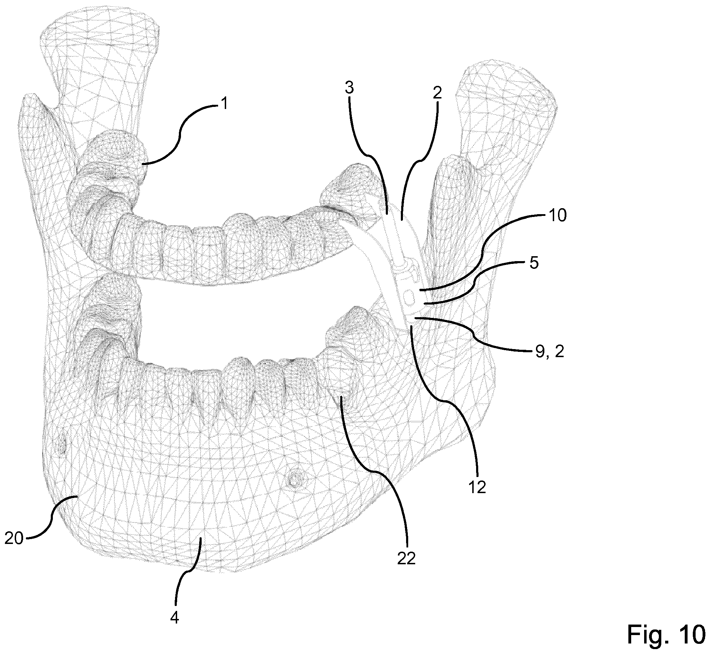

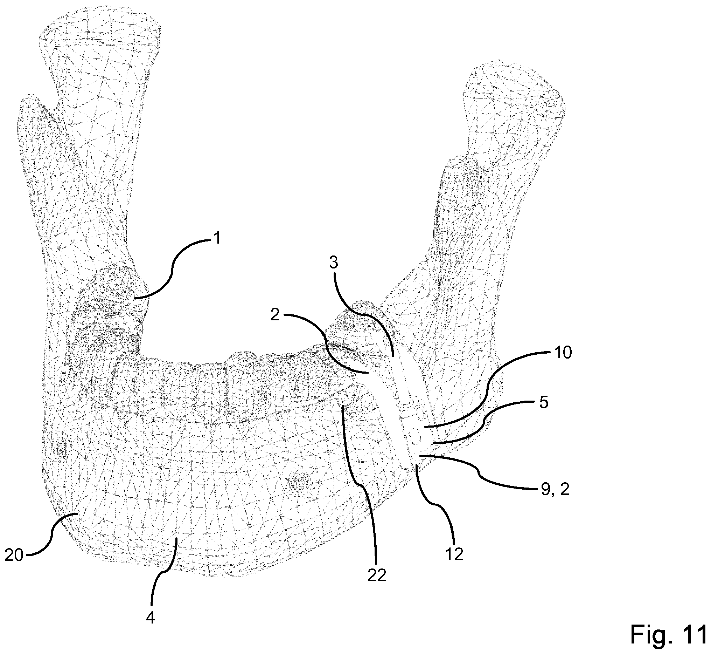

Bei dem in den Fig. 9 bis 11 dargestellten Ausführungsbeispiel weist das Führungsmittel 2 eine Aufnahme 9 für das Führungselement 11 des Kopfes 10 auf und ist mit der Schablone 1 verbunden. Die Schablone 1 ist form- bzw. kraftschlüssig an den Zähnen des dargestellten Unterkiefers 20 anordenbar. Durch die Ausgestaltung des Führungsmittels 2 wird die Hohlfräse 3 entlang des Knochens 4 geführt, so dass ein Knochensegment entnehmbar ist. Dabei ist denkbar, dass das Führungsmittel 2 lösbar oder unlösbar mit der Schablone 1 ausgebildet ist. Des Weiteren wird auf die voranstehende Figurenbeschreibung verwiesen, die analog für das Ausführungsbeispiel der Fig. 9 bis 11 gilt.

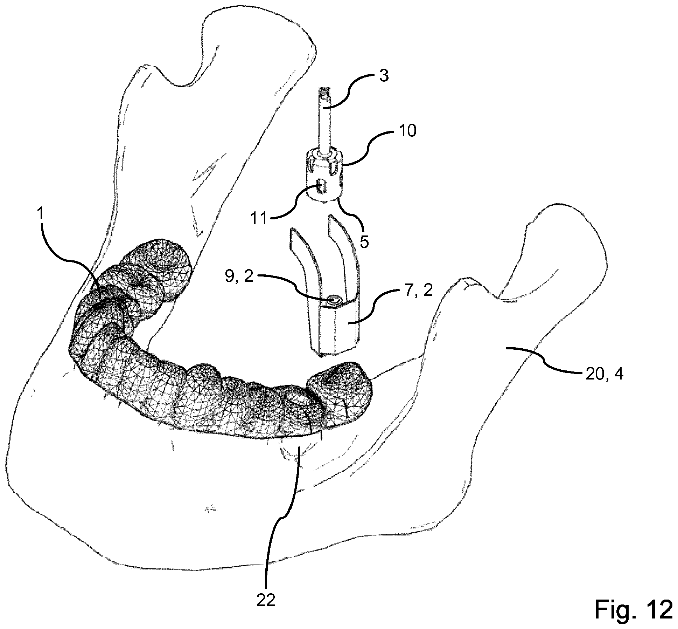

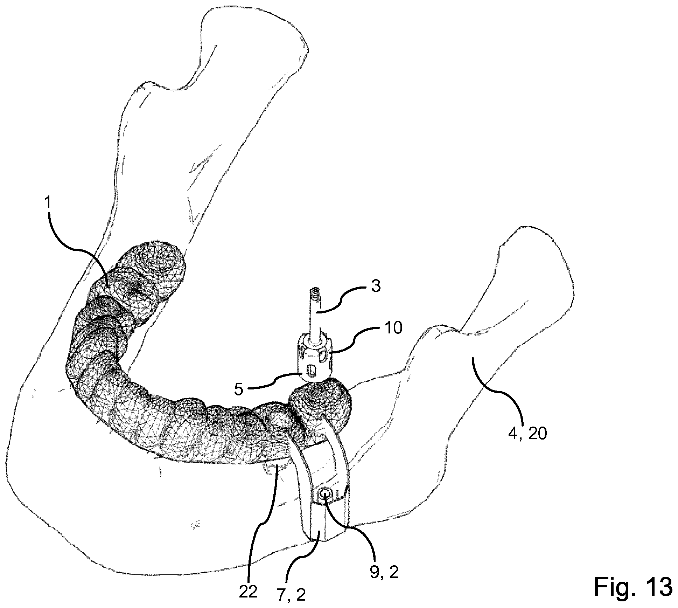

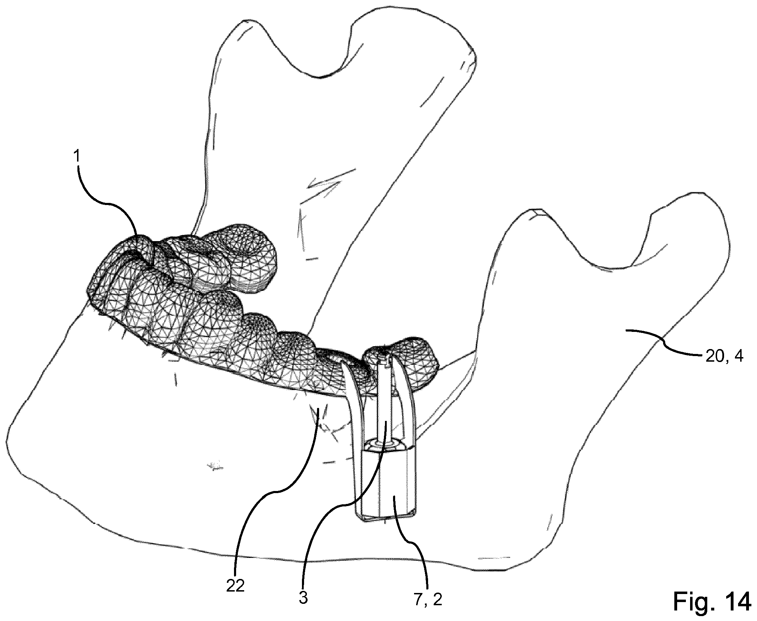

Das in den Fig. 12 bis 14 dargestellte Ausführungsbeispiel entspricht dem in den Fig. 9 bis 11 dargestellten Ausführungsbeispiel, wobei das Führungsmittel 2 neben der Aufnahme 9 zusätzlich als Führungshülse 7 ausgebildet ist. Die Führungshülse 7 weist dabei eine eckige Geometrie auf und dient insbesondere zum Schutz des umliegenden Gewebes. Des Weiteren sei darauf hingewiesen, dass die Führungshülse 7 an ihrem Boden 21 geschlossen ausgebildet sein kann. Insbesondere ist denkbar, dass das Führungsmittel 2 lösbar oder unlösbar mit der Schablone 1 ausgebildet ist.

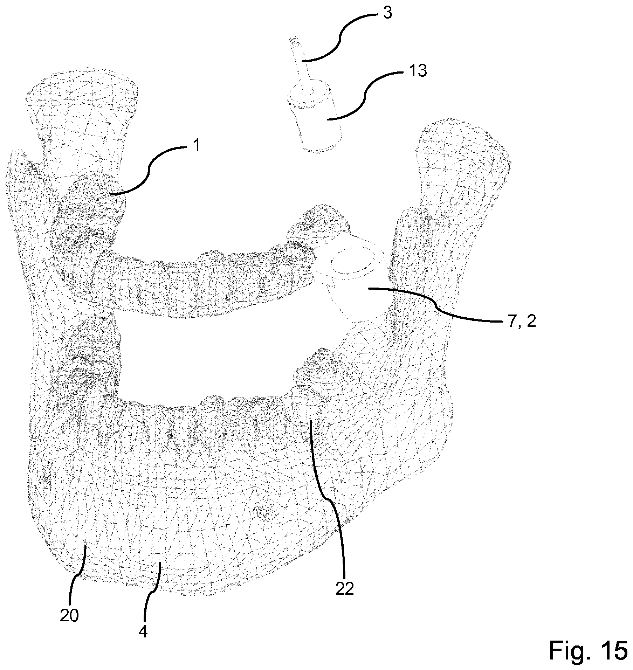

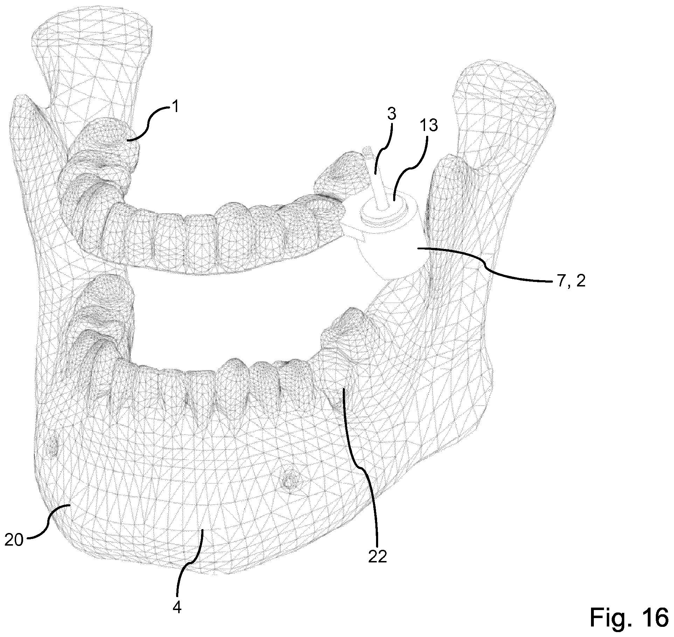

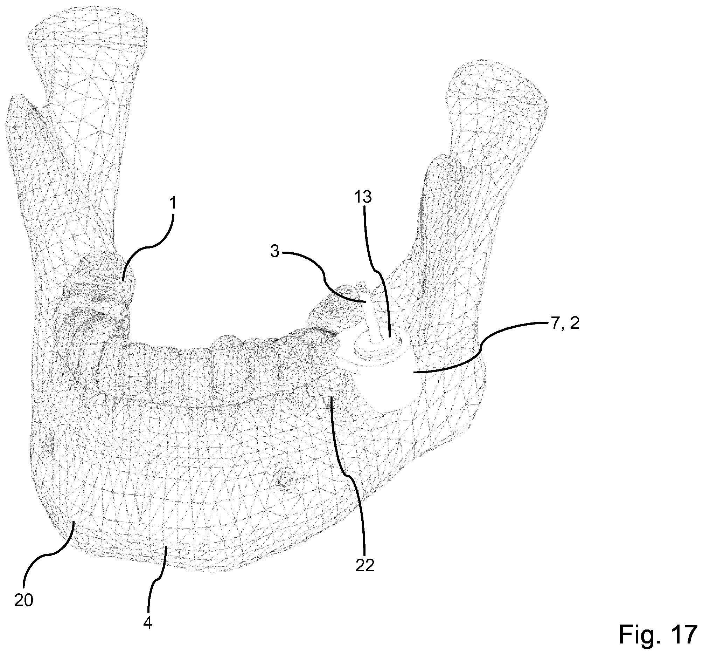

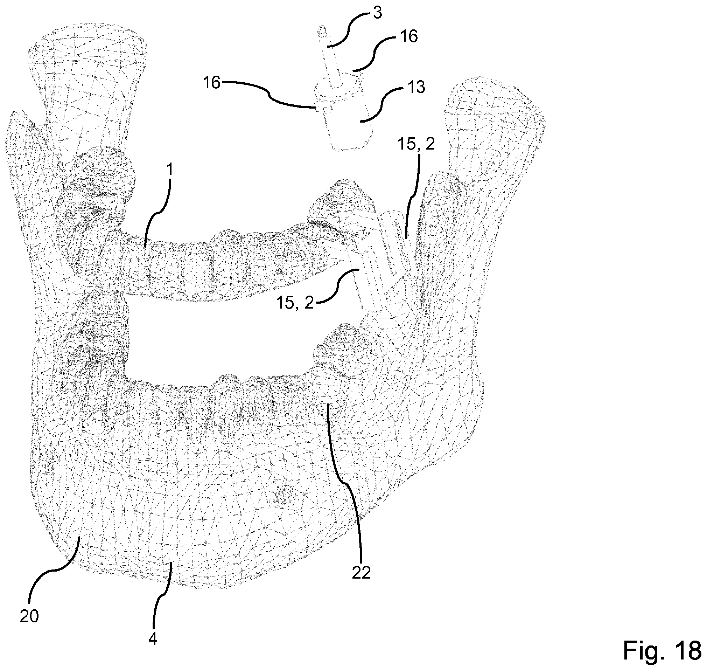

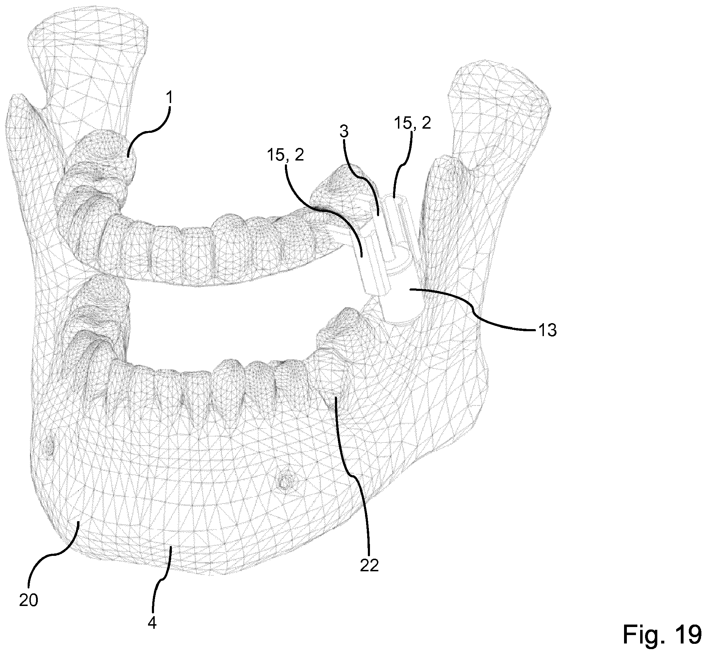

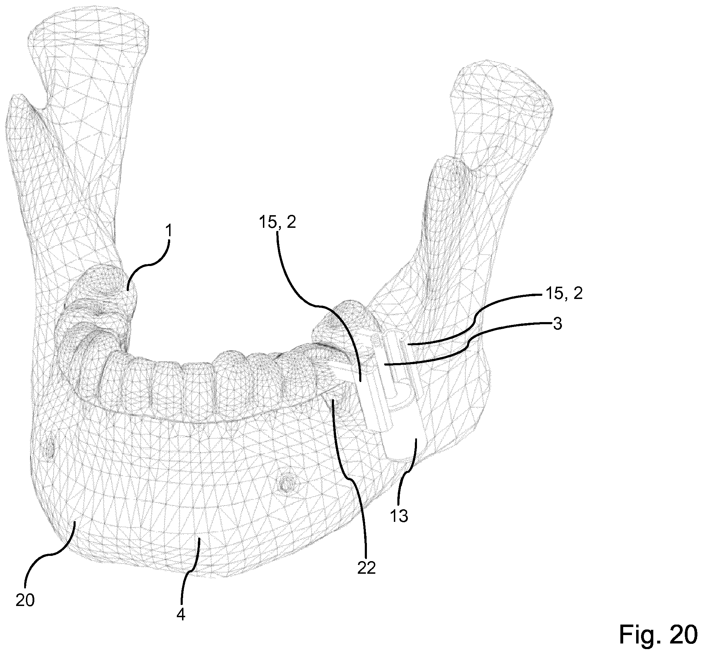

Das Ausführungsbeispiel gemäß Fig. 15 bis 17 entspricht im Wesentlichen dem Ausführungsbeispiel gemäß Fig. 3 und 4. Weiterhin entspricht das Ausführungsbeispiel gemäß Fig. 18 bis 20 im Wesentlichen der in den Fig. 5 und 6 gezeigten Vorrichtung. Zur Vermeidung von Wiederholungen wird an dieser Stelle auf die voranstehende Figurenbeschreibung verwiesen, wobei jeweils das Führungsmittel 2 lösbar oder unlösbar mit der Schablone 1 ausgebildet sein kann.

Die in den Ausführungsbeispielen der Fig. 1 bis 20 dargestellten Schablonen 1 können durch das offenbarte Verfahren hergestellt werden. Dabei wird zunächst ein dreidimensionales, bildgebendes Verfahren durchgeführt, um dreidimensionale Daten von dem Kieferknochen 4 bzw. der Entnahmestelle 6 und ggf. dem Knochendefizit 22 zu erhalten. Anhand der dreidimensionalen Daten wird ein dreidimensionales Computermodell des Kieferknochens 4 erstellt. Anhand des Computermodells lässt sich die Entnahmestelle 6 ermitteln, nämlich unter Berücksichtigung der patientenspezifischen Anatomie. Auf Grundlage des Computermodells wird somit festgelegt, wie die patientenspezifische Schablone 1 für den Eingriff ausgebildet sein muss, insbesondere wo bzw. in welchem Winkel das Führungsmittel 2 an der Schablone 1 ausgebildet sein müssen und wie diese zu dimensionieren sind. Dabei kann ggf. auch die Ausgestaltung des oder mehrerer Führungsmittel 2 und/oder. Somit kann auf Grundlage des dreidimensionalen Computermodells eine patientenspezifische Vorrichtung erzeugt werden. Dies kann beispielsweise im Rapid-Prototyping-Verfahren erfolgen.

Schließlich sei ausdrücklich darauf hingewiesen, dass die voranstehend beschriebenen Ausführungsbeispiele der erfindungsgemäßen Vorrichtung lediglich zur Erörterung der beanspruchten Lehre dienen, diese jedoch nicht auf die Ausführungsbeispiele einschränken.

### Bezugszeichenliste

- 1: Schablone
- 2: Führungsmittel
- 3: Hohlfräse
- 4: Kieferknochen
- 5: distaler Rand
- 6: Entnahmestelle
- 7: Führungshülse
- 8: Öffnung
- 9: Aufnahme
- 10: Kopf
- 11: Führungselement
- 12: Tiefenanschlag
- 13: Schutzeinrichtung
- 14: Distanzelement
- 15: Schienen (Führungsmittel)
- 16: Schienen (Schutzeinrichtung)
- 17: Bodenelement
- 18: Fixierelemente
- 19: Nut
- 20: Unterkiefer
- 21: Boden
- 22: Knochendefizit

## Patentansprüche

1. Vorrichtung zum Führen einer dentalchirurgischen Hohlfräse (3) bei einer Knochenentnahme, mit einer im Bereich einer Entnahmestelle (6) festlegbaren Schablone (1) und mindestens einem an der Schablone (1) angeordneten Führungsmittel (2), wobei das Führungsmittel (2) die Hohlfräse (3) derart führt, dass lediglich ein Teil des distalen Randes (5) der Hohlfräse (3) mit dem Knochen (4) in Kontakt bringbar ist und wobei die Hohlfräse (3) durch das Führungsmittel (2) seitlich am Knochen (4) entlangführbar ist, so dass ein kreissegmentförmiges Knochenstück auslösbar ist.

2. Vorrichtung nach Anspruch 1, **dadurch gekennzeichnet, dass** die Schablone (1) form- und/oder kraftschlüssig im Oberkiefer und/oder im Unterkiefer (20) festlegbar ist.

3. Vorrichtung nach Anspruch 1 oder 2, **dadurch gekennzeichnet, dass** das Führungsmittel (2) die Hohlfräse (3) derart umgibt, dass das die Entnahmestelle (6) umliegende Gewebe nicht mit der Hohlfräse (3) in Kontakt kommt.

4. Vorrichtung nach einem der Ansprüche 1 bis 3, **dadurch gekennzeichnet, dass** das Führungsmittel (2) als Führungshülse (7) zur Aufnahme der Hohlfräse (3) ausgebildet ist.

5. Vorrichtung nach einem der Ansprüche 1 bis 4, **dadurch gekennzeichnet, dass** das Führungsmittel (2) eine Aufnahme (9) für ein Führungselement (11) der Hohlfräse (3) aufweist.

6. Vorrichtung nach einem der Ansprüche 1 bis 5, **dadurch gekennzeichnet, dass** an dem Führungsmittel (2) ein Tiefenanschlag (12) für die Hohlfräse (3) ausgebildet ist.

7. Vorrichtung nach einem der Ansprüche 1 bis 6, **dadurch gekennzeichnet, dass** das Führungsmittel (2) lösbar mit der Schablone (1) verbunden ist.

8. Vorrichtung nach einem der Ansprüche 1 bis 6, **dadurch gekennzeichnet, dass** das Führungsmittel (2) einteilig mit der Schablone (1) ausgebildet ist bzw. ein fester Bestandteil der Schablone (1) ist.

9. Vorrichtung nach einem der Ansprüche 1 bis 8, **dadurch gekennzeichnet, dass** die Schablone (1) ein Haltemittel aufweist, um das entnommenen Knochenstück im Bereich eines aufzubauenden Knochendefizits (22) zu halten.

## Claims

1. Device for guiding a dental-surgical hollow mill (3) during a bone removal operation, having a template (1) which can be secured in the region of a removal location (6) and at least one guide means (2) which is arranged on the template (1), wherein the guide means (2) guides the hollow mill (3) in such a manner that only a portion of the distal edge (5) of the hollow mill (3) can be brought into contact with the bone (4) and wherein the hollow mill (3) can be guided by the guide means (2) laterally along the bone (4) so that a circular-segment-like bone piece can be released.

2. Device according to claim 1, **characterised in that** the template (1) can be secured in a positive-locking and/or non-positive-locking manner in the upper jaw and/or in the lower jaw (20).

3. Device according to claim 1 or claim 2, **characterised in that** the guide means (2) surrounds the hollow mill (3) in such a manner that the tissue which surrounds the removal location (6) does not come into contact with the hollow mill (3) .

4. Device according to any one of claims 1 to 3, **characterised in that** the guide means (2) is constructed as a guide sleeve (7) for receiving the hollow mill (3).

5. Device according to any one of claims 1 to 4, **characterised in that** the guide means (2) has a receiving member (9) for a guide element (11) of the hollow mill (3).

6. Device according to any one of claims 1 to 5, **characterised in that** a depth stop (12) for the hollow mill (3) is formed on the guide means (2).

7. Device according to any one of claims 1 to 6, **characterised in that** the guide means (2) is releasably connected to the template (1).

8. Device according to any one of claims 1 to 6, **characterised in that** the guide means (2) is constructed integrally with the template (1) or is a fixed component of the template (1).

9. Device according to any one of claims 1 to 8, **characterised in that** the template (1) has a retention means in order to retain the removed piece of bone in the region of a bone deficit (22) which is intended to be built up.

## Revendications

1. Dispositif de guidage d'une fraise creuse (3) de chirurgie dentaire lors d'un prélèvement d'os, avec un gabarit (1) pouvant être fixé dans la zone d'un emplacement de prélèvement (6) et avec au moins un moyen de guidage (2) disposé sur le gabarit (1), le moyen de guidage (2) guidant la fraise creuse (3) de telle sorte que seule une partie du bord (5) distal de la fraise creuse (3) peut être amenée en contact avec l'os (4), et la fraise creuse (3) pouvant être guidée sur le côté de l'os (4) par le moyen de guidage (2) de telle sorte qu'une partie osseuse en forme de segment de cercle peut être détachée.

2. Dispositif selon la revendication 1, **caractérisé en ce que** le gabarit (1) peut être fixé par liaison de forme et/ou de force dans la mâchoire supérieure et/ou dans la mâchoire inférieure (20).

3. Dispositif selon la revendication 1 ou 2, **caractérisé en ce que** le moyen de guidage (2) entoure la fraise creuse (3) de telle sorte que le tissu environnant l'emplacement de prélèvement (6) ne vient pas en contact avec la fraise creuse (3).

4. Dispositif selon l'une des revendications 1 à 3, **caractérisé en ce que** le moyen de guidage (2) est constitué en tant que manchon de guidage (7) destiné à recevoir la fraise creuse (3).

5. Dispositif selon l'une des revendications 1 à 4, **caractérisé en ce que** le moyen de guidage (2) comporte un logement (9) pour un élément de guidage (11) de la fraise creuse (3).

6. Dispositif selon l'une des revendications 1 à 5, **caractérisé en ce qu'**une butée de profondeur (12) pour la fraise creuse (3) est constituée sur le moyen de guidage (2).

7. Dispositif selon l'une des revendications 1 à 6, **caractérisé en ce que** le moyen de guidage (2) est raccordé de façon détachable au gabarit (1).

8. Dispositif selon l'une des revendications 1 à 6, **caractérisé en ce que** le moyen de guidage (2) est constitué d'un seul tenant avec le gabarit (1) ou respectivement fait partie intégrante du gabarit (1).

9. Dispositif selon l'une des revendications 1 à 8, **caractérisé en ce que** le gabarit (1) comporte un moyen de retenue pour retenir la partie osseuse dans la zone d'un déficit osseux (22) à constituer.
